# EUROPEAN PATENT SPECIFICATION

(11) **EP 4 280 974 B1**
(45) Date of publication and mention of the grant of the patent: **15.05.2024**
(21) Application number: 22829885.7
(22) Date of filing: 14.12.2022
(51) Int. Cl.: A61B 17/04, A61B 90/00

(54) **TISSUE CLOSURE DEVICE**
GEWEBEVERSCHLUSSVORRICHTUNG
DISPOSITIF DE FERMETURE DE TISSU

(30) Priority: 17.12.2021 CN 202111551922
(43) Date of publication of application: 29.11.2023
(73) Proprietor: Cilag GmbH International, 6300 Zug (CH)
(72) Inventor: ZHANG, Jie, Shanghai 200233 (CN); LIU, Qinglong, Shanghai 200233 (CN)
(74) Representative: van Wanrooij, Eva
(86) International application number: PCT/IB2022/062186
(87) International publication number: WO 2023/111879

(56) References cited:
- EP-A1- 3 563 775
- US-A1- 2016 345 965
- US-A1- 2017 238 915

## Description

### TECHNICAL FIELD

The present invention relates to the field of surgical instruments, and in particular to a tissue closure device.

### BACKGROUND OF THE INVENTION

For an existing tissue closure device in surgical instruments, generally, the body of the closure device is penetrated into a human or animal tissue and then a suture is carried by an entry needle into the tissue, after which, the suture is fixed on a distal end structure of the tissue closure device, followed by closure. However, in the process of the entry needle carrying the suture into the tissue, the entry needle is likely to interfere with the body of the closure device or the tissue, which may cause the suture to fall off the entry needle, preventing the suture from being accurately delivered to a predetermined position. And the existing tissue closure device usually cannot accurately place the suture at a predetermined depth.

Therefore, there is a need to provide a tissue closure device that at least partially addresses the aforementioned problems.

EP 3 563 775 A1 discloses a port site closure instrument which includes a pair of wings and a body having proximal, central, and distal portions. The proximal portion defines a proximal suture aperture and first and second needle apertures. The central portion defines third and fourth needle apertures. A first needle channel is defined through the body communicating the first and third needle apertures. A second needle channel is defined through the body communicating the second and fourth needle apertures. The distal portion defines distal suture apertures. A first suture channel is defined by the body communicating the proximal and distal suture apertures. The pair of wings is pivotally supported by the distal portion of the body. Each of the wings defines a suture slot such that a suture exiting one of the distal suture apertures and positioned in the suture slots is aligned with one of the first or second needle channels.

### SUMMARY

The present invention provides a tissue closure device, the body of which can be pre-loaded with a suture prior to entering a tissue, so as to carry the suture into the tissue. A depth locating mechanism is unfolded after a distal end of the device enters the tissue, such that the suture is tensioned in a predetermined position to be gripped by an entry needle. Such an arrangement omits a needle inserting process for carrying a suture into a tissue, simplifies the operation process while ensuring that the suture can be accurately arranged in a predetermined position, and ensures the reliability of gripping the suture.

The present invention provides a tissue closure device according to claim 1. Optional features are recited in the dependent claims.

### BRIEF DESCRIPTION OF THE DRAWINGS

To better understand the above and other objectives, features, advantages and functions of the present invention, reference may be made to preferred embodiments shown in the accompanying drawings. The same or similar reference numerals in the drawings refer to the same or similar components. It should be understood by those skilled in the art that the drawings are intended to schematically clarify preferred embodiments of the present invention, without any limitation on the scope of the present invention, and various components in the figures are not necessarily drawn to scale.
FIG. 1 is a schematic perspective view of a tissue closure device according to one preferred embodiment of the present invention;
FIG. 2 is an enlarged schematic perspective view of one wing in FIG. 1;
FIG. 3A is a left view of the wing in FIG. 1;
FIG. 3B is a top view of the wing in FIG. 2;
FIG. 4 is an elevation view of the tissue closure device in FIG. 1 with a suture loaded thereon;
FIG. 5 is a perspective view of a tissue closure device according to another preferred embodiment of the present invention;
FIG. 6 is an enlarged partial view of the portion A in FIG. 5;
FIG. 7 is an enlarged perspective view of one wing in FIG. 6;
FIG. 8 is an elevation view of the tissue closure device in FIG. 5 with a suture loaded thereon;
FIG. 9 is a left view of the tissue closure device in FIG. 5; and
FIG. 10 is an enlarged partial view of the portion B in FIG. 9.

### DETAILED DESCRIPTION

A tissue closure device according to the present invention will be described in detail below with reference to the drawings. The given below are merely preferred embodiments according to the present invention, and on the basis of the preferred embodiments, those skilled in the art can conceive of other modes capable of implementing the present invention, which also fall within the scope of the present invention.

The present invention provides a tissue closure device for a surgical procedure. First of all, it should be noted that the directional terms and position terms mentioned in the present invention should be understood as relative directions and relative positions. For example, the "axis direction," "axial direction," and the like mentioned in the present invention may be understood as the direction of the axis defined by the elongate body; the "radial," "circumferential," and the like are radial, circumferential and the like with respect to the axis; "distal end" and "distal" is the direction along the axial direction and away from an operator (e.g., a surgeon), where the direction of distally is shown by D in the drawings; the "proximal end" and "proximal" is the direction along the axial direction and close to the operator (e.g., the surgeon), where the direction of proximally is shown by P in the drawings.

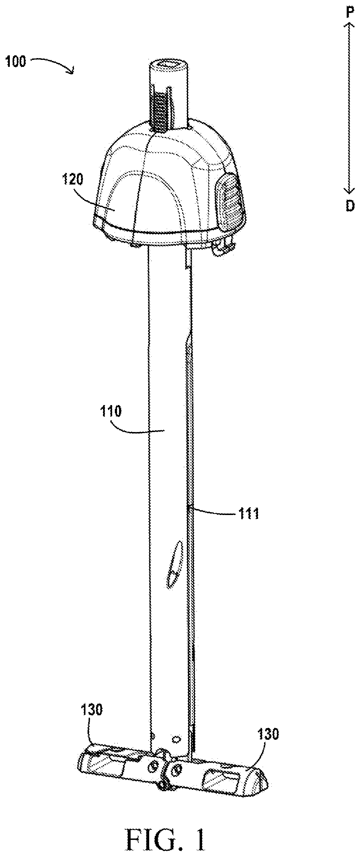

FIGS. 1-4 illustrate one preferred embodiment according to the present invention. Referring first to FIG. 1, a tissue closure device 100 includes an elongate body 110 having a longitudinal axis, an operating portion 120, and a depth limiting mechanism. The sidewall of the elongate body 110 is provided with a suture section receiving recess 111 extending parallel to the longitudinal axis, and the suture section receiving recess may also be disposed to extend from a proximal end to a distal end in a helical-like fashion about the sidewall of the elongate body. The operating portion 120 is disposed at a proximal end of the elongate body 110 and radially protrudes from the elongate body 110, and the operating portion 120 is provided with a proximal end engagement slot capable of clamping a suture 300.

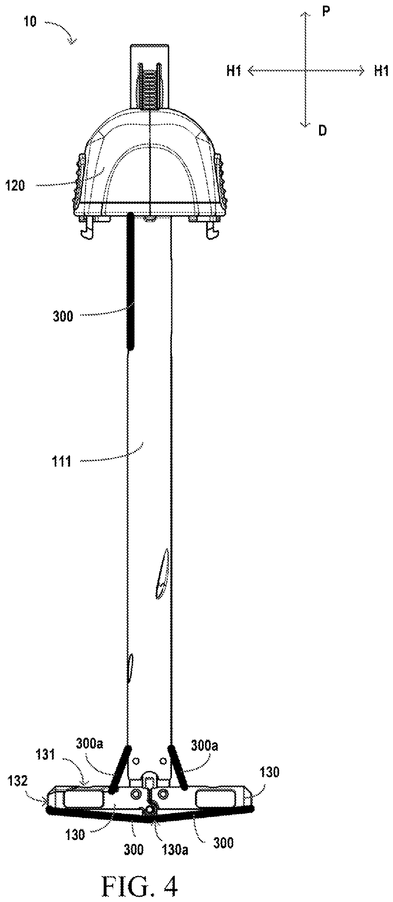

The depth limiting mechanism includes two wings 130 disposed about the longitudinal axis, and a proximal end of each wing 130 is pivotally connected to a distal end of the elongate body 110 about a pivot that is perpendicular to the longitudinal axis to allow movement of the wing 130 between a folded position and an unfolded position. When the two wings 130 are in the folded position, the two wings 130 together form an extension section of the elongate body 110; when the two wings 130 are in the unfolded position (i.e., the state shown in FIGS. 1 and 4), the two wings 130 extend radially outward relative to the elongate body 110 and together define a positioning plane at top surfaces of the two wings 130. The depth limiting mechanism is provided with a distal end engagement slot for clamping the suture 300, and the distal end engagement slot is at least partially formed on the wing 130.

The tissue closure device 100 is configured to allow the suture 300 to be tensioned between the proximal end engagement slot and the distal end engagement slot and received in the suture section receiving recess 111 prior to entry of the tissue closure device 100 into the tissue, and to cause the suture 300 to be stretched when the wing 130 is pivoted from the folded position to the unfolded position, so as to define a suture section 300a that is at least partially parallel to the positioning plane and can be gripped by an instrument (e.g., a needle). FIG. 4 illustrates a schematic view of the tissue closure device 100 with the suture 300 loaded thereon.

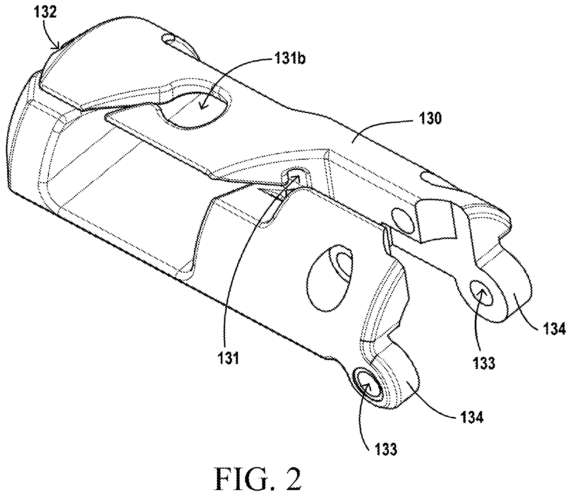

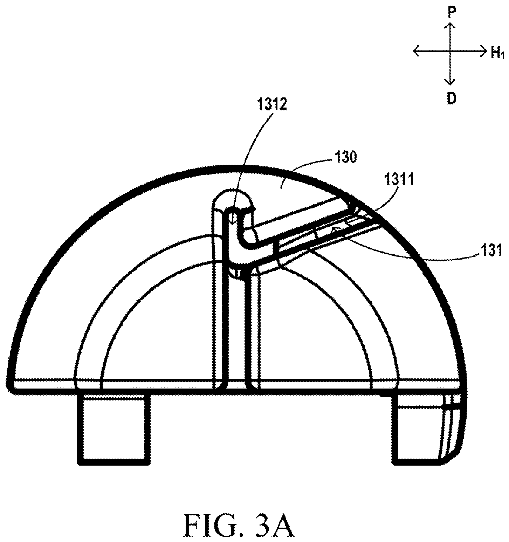

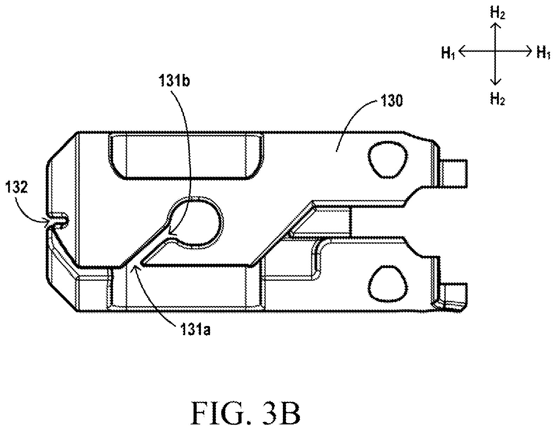

Preferably, with reference to FIGS. 2 and 3, the distal end engagement slot includes a first distal end engagement slot 131 and a second distal end engagement slot 132, and the first distal end engagement slot 131 and the second distal end engagement slot 132 are configured to allow the suture 300 to be gripped by the needle to disengage from the first distal end engagement slot 131 but remain engage with the second distal end engagement slot 132. In this embodiment, the first distal end engagement slot 131 and the second distal end engagement slot 132 are both disposed on the wing 130. And the first distal end engagement slot 131 is radially closer to the central axis than the second distal end engagement slot 132 when the wing 130 is in the unfolded position.

For example, inner walls of the first distal end engagement slot 131 and the second distal end engagement slot 132 may both be rigid. An opening direction of the first distal end engagement slot 131 is perpendicular to the longitudinal axis, and an included angle between the opening direction and a radial direction with respect to the axis is an acute angle, that is, the extension direction of the opening of the first distal end engagement slot 131 has a component extending along a first lateral direction H1 and also a component extending along a second lateral direction H2. The first lateral direction H1 and the second lateral direction H2 are perpendicular to each other and together define a plane perpendicular to the longitudinal axis. The first lateral direction H1 and the second lateral direction H2 may be parallel to two radial directions with respect to the longitudinal axis, respectively. As can be seen from FIGS. 2 and 3, the first distal end engagement slot 131 may be in communication with an open proximal end 131b, and the open proximal end 131b also has an opening 131a for outward withdrawal. The proximal end 131b is formed as an open, approximately circular opening, from where the instrument is allowed to grip the suture section.

With continued reference to FIGS. 2-3B, the second distal end engagement slot 132 may be formed at a radially outer end of the wing 130 and is disposed to open along the first lateral direction H1. The second distal end engagement slot 132 may be supported by silicone or other flexible materials (including elastic materials), such that the second distal end engagement slot 132 can elastically clamp the suture. Alternatively, the inner wall of the second distal end engagement slot 132 may also be rigid. As can also be seen from FIG. 2, the radially innermost side of the wing 130 that is close to the axis is provided with a pivot portion 134, and the pivot portion 134 is formed with a pivot hole 133. The pivot portion 134 protrudes distally relative to the body of the wing 130.

In the end view illustrated in FIG. 3A, it can be seen that the first distal end engagement slot 131 on the wing is formed as a hook-shaped slot, and the hook-shaped slot includes a vertical section 1312 extending parallel to the longitudinal axis and a section 1311 extending along the axial direction and the radial direction simultaneously. Such a unique suture slot design facilitates the insertion of the suture while ensuring that the suture can be released.

Referring to FIG. 4, when the suture 300 is pre-loaded on the tissue closure device 100, a proximal end of the suture 300 will be clamped on the proximal end engagement slot of the operating portion 120, and the suture 300 will extend to the distal end along the suture section receiving recess 111 on the elongate body 110 and be clamped by the first distal end engagement slot 131 and the second distal end engagement slot 132. In particular, the section of the suture 300 that is between the elongate body 110 and the first distal end engagement slot 131 forms a suture section 300a (which can be gripped from the circular opening 131b) which can be gripped by the needle. The most distal end portion of the suture 300 is tensioned between the respective second distal end engagement slots 132 of the two wings 130, and the middle of the portion rests against a distal end 130a of the two wings 130.

In use, the tissue closure device 100 can be pre-loaded with the suture 300 prior to entering a tissue, and the wings 130 are in the folded position prior to entering a tissue. The tissue closure device 100 loaded with the suture 300, with the wings 130 in the folded position, is extended into an incision in the tissue, and when the distal end of the tissue closure device 100 reaches a predetermined depth, the wings 130 can be unfolded by actuation of the operating portion 120. At this time, the suture 300 is also driven by the wings 130 to stretch, and the suture 300 is clamped in the first distal end engagement slot 131 and the second distal end engagement slot 132 respectively. At this time, the suture 300 has a suture section 300a which can be gripped by a needle.

Thereafter, a needle having a hook can be delivered into the tissue, and the hook can catch the suture section 300a of the suture 300 and pull the suture 300 so that the suture 300 is disengaged from the first distal end engagement slot 131. At this time, the suture 300 is still clamped by the second distal end engagement slot 132. Subsequently, the wings 130 can be pivoted back to the folded position by actuation of the operating portion 120 and moved proximally to exit the tissue. In the process, the suture 300 will be disengaged from the second distal end engagement slot 132, such that the distal end of the suture 300 is left within the tissue. After the tissue closure device 100 is removed from the tissue, the suture 300 can be sewn at the surface of the tissue.

In addition to the structures of the first distal end engagement slot and the second distal end engagement slot illustrated in FIGS. 1-4, there are also other possible arrangements for the first distal end engagement slot and the second distal end engagement slot disposed on the wings. In one embodiment, the first distal end engagement slot has a rigid inner slot wall and has an inner dimension greater than the diameter of the suture, and the second distal end engagement slot has an elastic inner slot wall and has an inner dimension less than the diameter of the suture so as to form an interference fit with the suture. For example, the first wing engagement slot is formed as an inclined slot extending distally and radially outward with respect to the central axis simultaneously, the first wing engagement slot having an inner dimension larger than the diameter of the suture, and/or the wing is provided with a silicone block, on which the second distal end engagement slot is formed on.

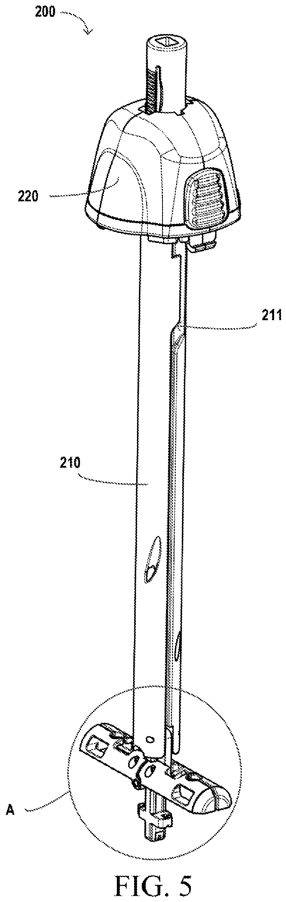

FIGS 5-10 illustrate another preferred embodiment according to the invention. Referring first to FIG. 5, a tissue closure device 200 includes an elongate body 210 having a longitudinal axis, an operating portion 220, and a depth limiting mechanism. The sidewall of the elongate body 210 is provided with a suture receiving recess 211 extending parallel to the longitudinal axis. The operating portion 220 is disposed at a proximal end of the elongate body 210 and radially protrudes from the elongate body 210, and the operating portion 220 is provided with a proximal end engagement slot capable of clamping a suture 300.

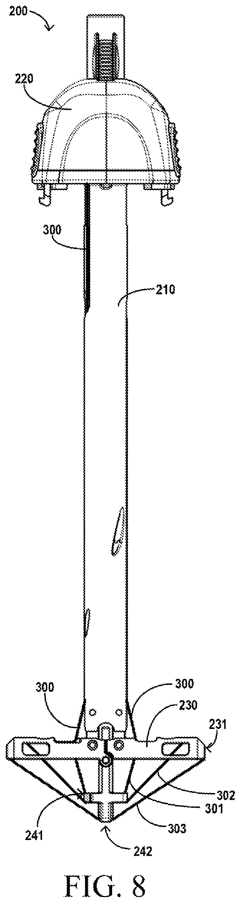

The depth limiting mechanism includes two wings 230 disposed about the longitudinal axis, and a proximal end of each wing 230 is pivotally connected to a distal end of the elongate body 210 about a pivot that is perpendicular to the longitudinal axis to allow movement of the wing 230 between a folded position and an unfolded position. When the two wings 230 are in the folded position, the two wings 230 together form an extension section of the elongate body 210; when the two wings 230 are in the unfolded position (i.e., the state shown in FIGS. 5 and 8), the two wings 230 extend radially outward relative to the elongate body 210 and together define a positioning plane at top surfaces of the two wings 230. The depth limiting mechanism is provided with a distal end engagement slot for clamping the suture 300, and the distal end engagement slot is at least partially formed on the wing 230.

The tissue closure device 200 is configured to allow the suture 300 to be tensioned between the proximal end engagement slot and the distal end engagement slot and received in the suture receiving recess 211 prior to entry of the tissue closure device 200 into the tissue, and to cause the suture 300 to be stretched when the wing 230 is pivoted from the folded position to the unfolded position, so as to define a suture section that is at least partially parallel to the positioning plane and can be gripped by an instrument. FIG. 8 illustrates a schematic view of the tissue closure device 200 with the suture 300 loaded thereon.

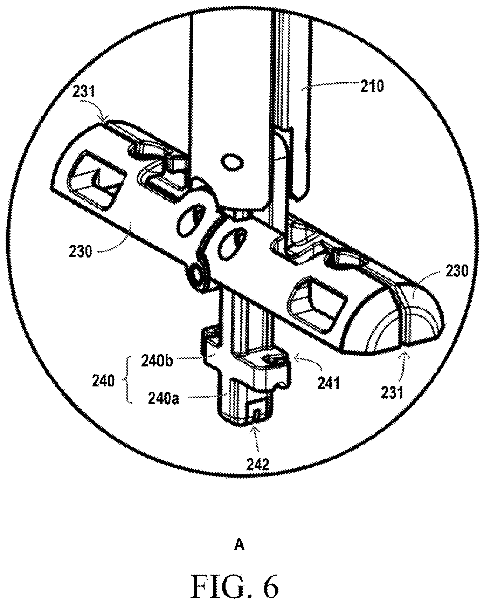

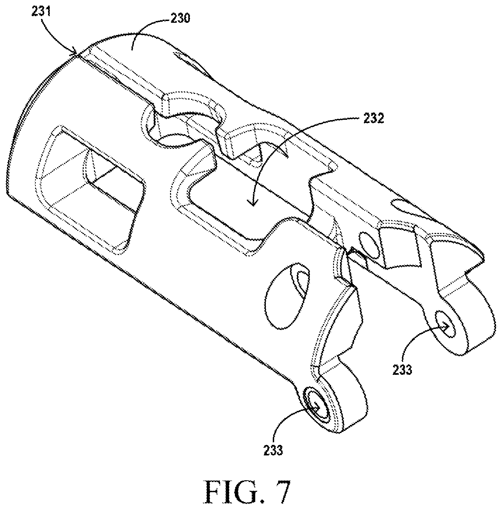

Referring to FIGS. 6 and 7, the depth limiting mechanism further includes a fixing block 240 fixedly connected to the distal end of the elongate body 210 and extending along the longitudinal axis, and the fixing block 240 is positioned such that the two wings 230 circumferentially surround the fixing block 240 when the two wings 230 are in the folded position, and the fixing block 240 is located distal to the two wings 230 when the two wings 230 are in the unfolded position. A first distal end engagement slot 241 is formed on the fixing block 240, and a second distal end engagement slot 231 is formed on the two wings 230. A hole 233 for receiving a pivot is formed on the wing 230.

Preferably, the fixing block 240 includes a longitudinal portion 240a extending along the longitudinal axis and a transverse portion 240b perpendicular to the longitudinal axis, and the longitudinal portion 240a and the transverse portion 240b together form a cruciform unit, where the first distal end engagement slot 241 is formed at two ends of the transverse portion 240b. The second distal end engagement slot 231 is formed at a distal end of each wing 230 (the distal end can be understood as a distal end when the wing 230 is in the folded position).

Further, a distal end of the longitudinal portion 240a of the fixing block 240 is also provided with a third distal end engagement slot 242, and the third distal end engagement slot 242 is configured such that the suture 300 remains engage with the third engagement slot 242 when the suture 300 is gripped by a needle. In this embodiment, the second distal end engagement slot 231 and the third distal end engagement slot 242 are both made of silicone which defines a slot dimension that is less than the width of the suture 300 so that the second distal end engagement slot 231 and the third distal end engagement slot 242 elastically retain the suture 300 in an interference fit manner.

Preferably, referring to FIG. 7, each wing 230 is provided with a window 232, and the window 232 is positioned to allow the suture 300 to extend distally through the window 232 from the suture receiving recess 211 of the elongate body 210 to the first distal end engagement slot 241.

Referring to FIG. 8, a first portion 301 of the suture 300 is tensioned between the elongate body 210 and the first distal end engagement slot 241; a second portion 302 is tensioned between the first distal end engagement slot 241 and the second distal end engagement slot 231; and a third portion 303 is tensioned between the second distal end engagement slot 231 and the third distal end engagement slot 242. In this embodiment, the second portion 302 of the suture 300 is a suture section which can be gripped by the needle.

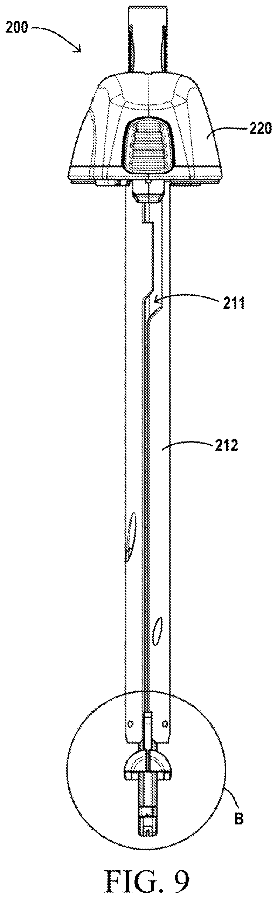

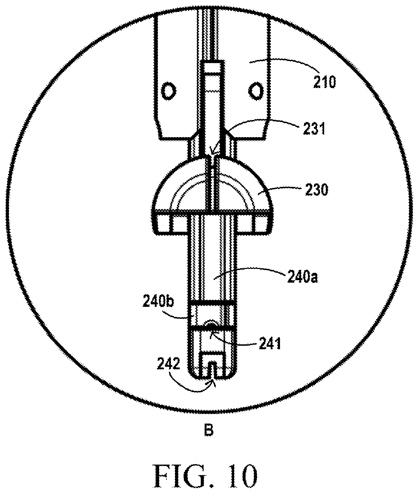

Referring to FIGS. 9 and 10, it can be more clearly seen that the first distal end engagement slot 241 is a shallow slot formed on a distal face at a radially outer end of a transverse portion 242b, the second distal end engagement slot 231 is formed as a slot axially penetrating on a radially outer end face of the wing 230, and the third distal end engagement slot 242 is formed on a distal face of a longitudinal portion 240a and has a greater depth than the first distal end engagement slot 241. Such an arrangement facilitates pre-loading of a suture, and can ensure that the suture is not easy to fall off when the suture is tensioned and released.

In use, the tissue closure device 200 can be pre-loaded with the suture 300 prior to entering a tissue, and the wings 230 are in the folded position prior to entering a tissue. The tissue closure device 200 loaded with the suture 300, with the wings 230 in the folded position, is extended into an incision in the tissue, and when the distal end of the tissue closure device 200 reaches a predetermined depth, the wings 230 can be unfolded by actuation of the operating portion 220. At this time, the suture 300 is also driven by the wings 230 to stretch, and the suture 300 is clamped by the first distal end engagement slot 241, the second distal end engagement slot 231 and the third distal end engagement slot 242 respectively. At this time, the suture 300 has a suture section (the second portion 302) which can be gripped by the needle.

Thereafter, a needle having a hook can be delivered into the tissue, and the hook can catch the suture section of the suture 300 and pull the suture 300 so that the suture 300 is disengaged from the first distal end engagement slot 241. At this time, the suture 300 is still clamped by the second distal end engagement slot 231 and the third distal end engagement slot 242. Subsequently, the wings 230 can be pivoted back to the folded position by actuation of the operating portion 220 and moved proximally to exit the tissue. In the process, the suture 300 will be disengaged from the second distal end engagement slot 231 and the third distal end engagement slot 242, such that the distal end of the suture 300 is left within the tissue. After the tissue closure device 200 is removed from the tissue, the suture 300 can be sewn at the surface of the tissue.

While the number of the wings in the embodiments illustrated in FIGS. 1-8 is only two, in other embodiments not shown, three or more wings may be provided, and the three or more wings may be arranged about the longitudinal axis at equal intervals, that is, any pair of adjacent wings has the same included angle.

The tissue closure device of the present invention can be pre-loaded with a suture prior to entering a tissue, to carry the suture into the tissue. A depth locating mechanism is unfolded after a distal end of the device enters the tissue, such that the suture is tensioned in a predetermined position to be gripped by an entry needle. Such an arrangement omits a needle inserting process for carrying a suture into a tissue, and simplifies the operation process while ensuring that the suture can be accurately arranged in a predetermined position without falling off in an undesired position.

From the above, those skilled in the art will readily recognize that alternative structures of the presently disclosed structures may be used as feasible alternative embodiments, and the presently disclosed embodiments may be combined to create new embodiments, which may also fall within the scope of the appended claims.

## Claims

1. A tissue closure device, the tissue closure device (100, 200) comprising:
an elongate body (110, 210) having a longitudinal axis, a sidewall of the elongate body being provided with a suture receiving recess (111, 211) extending from a proximal end to a distal end;
an operating portion (120, 220) disposed at the proximal end of the elongate body (110, 210);
a depth limiting mechanism comprising at least two wings (130, 230) disposed about the longitudinal axis, a proximal end of each wing (130, 230) being pivotally connected to the distal end of the elongate body (110, 220) about a pivot that is perpendicular to the longitudinal axis to allow movement of the wing (130, 230) between a folded position and an unfolded position, wherein the at least two wings (130, 230) together form an extension section of the elongate body (110, 210) when the at least two wings (130, 230) are in the folded position; and the at least two wings (130, 230) extend radially outward relative to the elongate body (110, 210) and together define a positioning plane at top surfaces of the at least two wings (130, 230) when the at least two wings (130, 230) are in the unfolded position, and the depth limiting mechanism is provided with a distal end engagement slot (131, 132, 231, 241) for clamping the suture, the distal end engagement (131, 132, 231, 241) slot being at least partially formed on the wing (130, 230),
**characterized in that** the operating portion (120, 220) is provided with a proximal end engagement slot capable of clamping a suture (300), wherein the tissue closure device is configured to allow the suture (300) to be retained between the proximal end engagement slot and the distal end engagement slot (131, 132, 231, 241) and received in the suture receiving recess (111, 211) prior to entry of the tissue closure device into a tissue, and to cause the suture (300) to be stretched when the at least two wings (130, 230) are pivoted from the folded position to the unfolded position, so as to define a suture section (300a, 302) that can be gripped by an instrument,
wherein the distal end engagement slot comprises a first distal end engagement slot (131, 241) and a second distal end engagement slot (132, 231), wherein the first distal end engagement slot and the second distal end engagement slot are arranged such that the defined suture section (300a, 302) that can be gripped by an instrument is positioned therebetween, the first distal end engagement slot (131, 241) and the second distal end engagement slot (132, 231) being configured to enable the suture (300) to disengage from the first distal end engagement slot (131, 241) but remain engaged with the second distal end engagement slot (132. 231) when gripped by the instrument.

2. The tissue closure device according to claim 1, wherein the first distal end engagement slot has a rigid inner slot wall and has an inner dimension greater than the diameter of the suture, and the second distal end engagement slot has an elastic inner slot wall and has an inner dimension less than the diameter of the suture so as to form an interference fit with the suture.

3. The tissue closure device according to claim 1 or claim 2, wherein both the first distal end engagement slot and the second distal end engagement slot are disposed on the wing.

4. The tissue closure device according to claim 3, wherein the first wing engagement slot is formed as a hook-shaped slot comprising an inclined slot extending distally and radially outward simultaneously, the first wing engagement slot having an inner dimension greater than the diameter of the suture.

5. The tissue closure device according to claim 3 or claim 4, wherein the wing is provided with a feature made of a flexible material, the second distal end engagement slot being formed in the feature.

6. The tissue closure device according to any one of claims 3-5, wherein the first distal end engagement slot and the second distal end engagement slot are positioned such that the first distal end engagement slot is radially closer to the central axis than the second distal end engagement slot when the wing is in the unfolded position.

7. The tissue closure device according to any one of claims 1-6, wherein inner walls of both the first distal end engagement slot (131) and the second distal end engagement slot (132) are rigid, wherein:
an opening direction of the first distal end engagement slot (131) is perpendicular to the longitudinal axis and an included angle between the opening direction and a radial direction with respect to the axis is an acute angle, a proximal end (131b) of the first distal end engagement slot being open; and
an opening direction of the second distal end engagement slot (132) is at least partially transverse to the first distal end engagement slot, and the second distal end engagement slot is positioned at a radially outer end of the wing.

8. The tissue closure device according to any one of claims 1-7, wherein the depth limiting mechanism further comprises a fixing block (240) fixedly connected to the distal end of the elongate body and extending along the longitudinal axis, the fixing block being positioned such that: the at least two wings circumferentially surround the fixing block when the at least two wings are in the folded position, and the fixing block is located distal to a junction of the at least two wings when the at least two wings are in the unfolded position,
wherein the first distal end engagement slot (241) is formed on the fixing block, and the second distal end engagement slot (231) is formed on the at least two wings.

9. The tissue closure device according to claim 8, wherein the fixing block comprises a longitudinal portion (240a) extending along the longitudinal axis and a transverse portion (240b) perpendicular to the longitudinal axis, the longitudinal portion and the transverse portion together forming a cruciform unit, wherein the first distal end engagement slot (241) is formed at two ends of the transverse portion (240a).

10. The tissue closure device according to claim 9, wherein the first distal end engagement slot (241) is formed on a distal face at a radially outer end of the transverse portion.

11. The tissue closure device according to any one of claims 8 - 10, wherein the second distal end engagement slot (231) is formed at a distal end of each of the wings.

12. The tissue closure device according to any one of claims 8 - 11, wherein a distal end of the longitudinal portion of the fixing block is further provided with a third distal end engagement slot (242), the third distal end engagement slot being configured such that the suture remains engaged with the third engagement slot when gripped by the instrument.

13. The tissue closure device according to any one of claims 7 - 12, wherein each wing is provided with a window (232), the window being positioned to allow the suture to extend distally through the window from the suture receiving recess of the elongate body to the first distal end engagement slot.

## Patentansprüche

1. Gewebeverschlussvorrichtung, die Gewebeverschlussvorrichtung (100, 200), umfassend:
einen länglichen Körper (110, 210), der eine Längsachse aufweist, wobei eine Seitenwand des länglichen Körpers mit einer Nahtaufnahmeaussparung (111, 211) versehen ist, die sich von einem proximalen Ende zu einem distalen Ende erstreckt;
einen Betätigungsabschnitt (120, 220), der an dem proximalen Ende des länglichen Körpers (110, 210) eingerichtet ist;
einen Tiefenbegrenzungsmechanismus, umfassend mindestens zwei Flügel (130, 230), die um die Längsachse herum eingerichtet sind, wobei ein proximales Ende jedes Flügels (130, 230) mit dem distalen Ende des länglichen Körpers (110, 220) um einen Drehpunkt, der senkrecht zu der Längsachse ist, schwenkbar verbunden ist, um eine Bewegung des Flügels (130, 230) zwischen einer eingeklappten Position und einer aufgeklappten Position zu ermöglichen, wobei die mindestens zwei Flügel (130, 230) zusammen einen Verlängerungsbereich des länglichen Körpers (110, 210) ausbilden, wenn die mindestens zwei Flügel (130, 230) in der eingeklappten Position sind; und sich die mindestens zwei Flügel (130, 230) relativ zu dem länglichen Körper (110, 210) radial nach außen erstrecken und zusammen eine Positionierungsebene an oberen Oberflächen der mindestens zwei Flügel (130, 230) definieren, wenn die mindestens zwei Flügel (130, 230) in der aufgeklappten Position sind, und der Tiefenbegrenzungsmechanismus mit einem distalen Endeingriffsschlitz (131, 132, 231, 241) zum Klemmen des Fadens versehen ist, wobei der distale Endeingriffs(131, 132, 231, 241)-Schlitz mindestens teilweise auf dem Flügel (130, 230) ausgebildet ist,
**dadurch gekennzeichnet, dass** der Betätigungsabschnitt (120, 220) mit einem proximalen Endeingriffsschlitz versehen ist, der in der Lage ist, einen Faden (300) zu klemmen, wobei die Gewebeverschlussvorrichtung konfiguriert ist, um zu ermöglichen, dass der Faden (300) zwischen dem proximalen Endeingriffsschlitz und dem distalen Endeingriffsschlitz (131, 132, 231, 241) gehalten und in der Nahtaufnahmeaussparung (111, 211) vor dem Eintritt der Gewebeverschlussvorrichtung in ein Gewebe aufgenommen wird, und um zu bewirken, dass der Faden (300) gedehnt wird, wenn die mindestens zwei Flügel (130, 230) von der eingeklappten Position in die aufgeklappte Position geschwenkt werden, um einen Nahtbereich (300a, 302) zu definieren, der von einem Instrument gegriffen werden kann,
wobei der distale Endeingriffsschlitz einen ersten distalen Endeingriffsschlitz (131, 241) und einen zweiten distalen Endeingriffsschlitz (132, 231) umfasst, wobei der erste distale Endeingriffsschlitz und der zweite distale Endeingriffsschlitz derart angeordnet sind, dass der definierte Nahtbereich (300a, 302), der von einem Instrument gegriffen werden kann, dazwischen positioniert ist, wobei der erste distale Endeingriffsschlitz (131, 241) und der zweite distale Endeingriffsschlitz (132, 231) konfiguriert sind, um zu ermöglichen, dass sich der Faden (300) von dem ersten distalen Endeingriffsschlitz (131, 241) löst, aber mit dem zweiten distalen Endeingriffsschlitz (132, 231) in Eingriff bleibt, wenn dieser von dem Instrument gegriffen wird.

2. Gewebeverschlussvorrichtung nach Anspruch 1, wobei der erste distale Endeingriffsschlitz eine starre Innenschlitzwand aufweist und eine Innenabmessung aufweist, die größer als der Durchmesser des Fadens ist, und der zweite distale Endeingriffsschlitz eine elastische Innenschlitzwand aufweist und eine Innenabmessung aufweist, die kleiner als der Durchmesser des Fadens ist, um eine Presspassung mit dem Faden auszubilden.

3. Gewebeverschlussvorrichtung nach Anspruch 1 oder 2, wobei sowohl der erste distale Endeingriffsschlitz als auch der zweite distale Endeingriffsschlitz an dem Flügel eingerichtet sind.

4. Gewebeverschlussvorrichtung nach Anspruch 3, wobei der erste Flügeleingriffsschlitz als hakenförmiger Schlitz ausgebildet ist, umfassend einen geneigten Schlitz, der sich gleichzeitig distal und radial nach außen erstreckt, wobei der erste Flügeleingriffsschlitz eine Innenabmessung aufweist, die größer als der Durchmesser des Fadens ist.

5. Gewebeverschlussvorrichtung nach Anspruch 3 oder 4, wobei der Flügel mit einem Merkmal versehen ist, das aus einem flexiblen Material hergestellt ist, wobei der zweite distale Endeingriffsschlitz in dem Merkmal ausgebildet ist.

6. Gewebeverschlussvorrichtung nach einem der Ansprüche 3 bis 5, wobei der erste distale Endeingriffsschlitz und der zweite distale Endeingriffsschlitz derart positioniert sind, dass der erste distale Endeingriffsschlitz radial näher an der Mittelachse als der zweite distale Endeingriffsschlitz liegt, wenn der Flügel in der ausgeklappten Position ist.

7. Gewebeverschlussvorrichtung nach einem der Ansprüche 1 bis 6, wobei Innenwände sowohl des ersten distalen Endeingriffsschlitzes (131) als auch des zweiten distalen Endeingriffsschlitzes (132) starr sind, wobei:
eine Öffnungsrichtung des ersten distalen Endeingriffsschlitzes (131) senkrecht zu der Längsachse ist und ein eingeschlossener Winkel zwischen der Öffnungsrichtung und einer radialen Richtung in Bezug auf die Achse ein spitzer Winkel ist, wobei ein proximales Ende (131b) des ersten distalen Endeingriffsschlitzes offen ist; und
eine Öffnungsrichtung des zweiten distalen Endeingriffsschlitzes (132) mindestens teilweise quer zu dem ersten distalen Endeingriffsschlitz ist, und der zweite distale Endeingriffsschlitz an einem radial äußeren Ende des Flügels positioniert ist.

8. Gewebeverschlussvorrichtung nach einem der Ansprüche 1 bis 7, wobei der Tiefenbegrenzungsmechanismus ferner einen Befestigungsblock (240) umfasst, der mit dem distalen Ende des länglichen Körpers fest verbunden ist und sich entlang der Längsachse erstreckt, wobei der Befestigungsblock derart positioniert ist, dass: die mindestens zwei Flügel den Befestigungsblock in Umfangsrichtung umgeben, wenn die mindestens zwei Flügel in der eingeklappten Position sind, und sich der Befestigungsblock distal zu einer Verbindungsstelle der mindestens zwei Flügel befindet, wenn die mindestens zwei Flügel in der aufgeklappten Position sind,
wobei der erste distale Endeingriffsschlitz (241) auf dem Befestigungsblock ausgebildet ist, und der zweite distale Endeingriffsschlitz (231) auf den mindestens zwei Flügeln ausgebildet ist.

9. Gewebeverschlussvorrichtung nach Anspruch 8, wobei der Befestigungsblock einen Längsabschnitt (240a), der sich entlang der Längsachse erstreckt, und einen Querabschnitt (240b) senkrecht zu der Längsachse umfasst, wobei der Längsabschnitt und der Querabschnitt zusammen eine kreuzförmige Einheit ausbilden, wobei der erste distale Endeingriffsschlitz (241) an zwei Enden des Querabschnitts (240a) ausgebildet ist.

10. Gewebeverschlussvorrichtung nach Anspruch 9, wobei der erste distale Endeingriffsschlitz (241) auf einer distalen Fläche an einem radial äußeren Ende des Querabschnitts ausgebildet ist.

11. Gewebeverschlussvorrichtung nach einem der Ansprüche 8 bis 10, wobei der zweite distale Endeingriffsschlitz (231) an einem distalen Ende jedes der Flügel ausgebildet ist.

12. Gewebeverschlussvorrichtung nach einem der Ansprüche 8 bis 11, wobei ein distales Ende des Längsabschnitts des Befestigungsblocks ferner mit einem dritten distalen Endeingriffsschlitz (242) versehen ist, wobei der dritte distale Endeingriffsschlitz derart konfiguriert ist, dass der Faden mit dem dritten Eingriffsschlitz in Eingriff bleibt, wenn er von dem Instrument gegriffen wird.

13. Gewebeverschlussvorrichtung nach einem der Ansprüche 7 bis 12, wobei jeder Flügel mit einem Fenster (232) versehen ist, wobei das Fenster positioniert ist, um zu ermöglichen, dass sich der Faden distal durch das Fenster von der Nahtaufnahmeaussparung des länglichen Körpers zu dem ersten distalen Endeingriffsschlitz erstreckt.

## Revendications

1. Dispositif de fermeture de tissu, le dispositif de fermeture de tissu (100, 200) comprenant :
un corps allongé (110, 210) ayant un axe longitudinal, une paroi latérale du corps allongé étant pourvue d'un évidement de réception de suture (111, 211) s'étendant d'une extrémité proximale à une extrémité distale ;
une partie d'actionnement (120, 220) disposée au niveau de l'extrémité proximale du corps allongé (110, 210) ;
un mécanisme de limitation de profondeur comprenant au moins deux ailes (130, 230) disposées autour de l'axe longitudinal, une extrémité proximale de chaque aile (130, 230) étant reliée de manière pivotante à l'extrémité distale du corps allongé (110, 220) autour d'un pivot qui est perpendiculaire à l'axe longitudinal pour permettre un mouvement de l'aile (130, 230) entre une position pliée et une position dépliée, dans lequel les au moins deux ailes (130, 230) forment ensemble une section d'extension du corps allongé (110, 210) lorsque les au moins deux ailes (130, 230) sont dans la position pliée ; et les au moins deux ailes (130, 230) s'étendent radialement vers l'extérieur par rapport au corps allongé (110, 210) et définissent ensemble un plan de positionnement au niveau de surfaces supérieures des au moins deux ailes (130, 230) lorsque les au moins deux ailes (130, 230) sont dans la position dépliée, et le mécanisme de limitation de profondeur est pourvu d'une fente de mise en prise d'extrémité distale (131, 132, 231, 241) permettant de serrer la suture, la fente de mise en prise d'extrémité distale (131, 132, 231, 241) étant au moins partiellement formée sur l'aile (130, 230),
**caractérisé en ce que** la partie d'actionnement (120, 220) est pourvue d'une fente de mise en prise d'extrémité proximale capable de serrer une suture (300), dans lequel le dispositif de fermeture de tissu est conçu pour permettre à la suture (300) d'être retenue entre la fente de mise en prise d'extrémité proximale et la fente de mise en prise d'extrémité distale (131, 132, 231, 241) et reçue dans l'évidement de réception de suture (111, 211) avant l'entrée du dispositif de fermeture de tissu dans un tissu, et pour amener la suture (300) à être étirée lorsque les au moins deux ailes (130, 230) sont amenées à pivoter de la position pliée à la position dépliée, de manière à définir une section de suture (300a, 302) qui peut être saisie par un instrument,
dans lequel la fente de mise en prise d'extrémité distale comprend une première fente de mise en prise d'extrémité distale (131, 241) et une deuxième fente de mise en prise d'extrémité distale (132, 231), dans lequel la première fente de mise en prise d'extrémité distale et la deuxième fente de mise en prise d'extrémité distale sont agencées de telle sorte que la section de suture définie (300a, 302) qui peut être saisie par un instrument est positionnée entre elles, la première fente de mise en prise d'extrémité distale (131, 241) et la deuxième fente de mise en prise d'extrémité distale (132, 231) étant conçues pour permettre à la suture (300) de se désolidariser de la première fente de mise en prise d'extrémité distale (131, 241) mais de rester en prise avec la deuxième fente de mise en prise d'extrémité distale (132. 231) lorsqu'elle est saisie par l'instrument.

2. Dispositif de fermeture de tissu selon la revendication 1, dans lequel la première fente de mise en prise d'extrémité distale a une paroi de fente interne rigide et a une dimension interne supérieure au diamètre de la suture, et la deuxième fente de mise en prise d'extrémité distale a une paroi de fente interne élastique et a une dimension interne inférieure au diamètre de la suture de manière à former un ajustement serré avec la suture.

3. Dispositif de fermeture de tissu selon la revendication 1 ou la revendication 2, dans lequel à la fois la première fente de mise en prise d'extrémité distale et la deuxième fente de mise en prise d'extrémité distale sont disposées sur l'aile.

4. Dispositif de fermeture de tissu selon la revendication 3, dans lequel la première fente de mise en prise d'aile est formée comme une fente en forme de crochet comprenant une fente inclinée s'étendant de manière distale et radiale vers l'extérieur simultanément, la première fente de mise en prise d'aile ayant une dimension interne supérieure au diamètre de la suture.

5. Dispositif de fermeture de tissu selon la revendication 3 ou la revendication 4, dans lequel l'aile est pourvue d'une caractéristique faite d'un matériau souple, la deuxième fente de mise en prise d'extrémité distale étant formée dans la caractéristique.

6. Dispositif de fermeture de tissu selon l'une quelconque des revendications 3 à 5, dans lequel la première fente de mise en prise d'extrémité distale et la deuxième fente de mise en prise d'extrémité distale sont positionnées de telle sorte que la première fente de mise en prise d'extrémité distale est radialement plus proche de l'axe central que la deuxième fente de mise en prise d'extrémité distale lorsque l'aile est dans la position dépliée.

7. Dispositif de fermeture de tissu selon l'une quelconque des revendications 1 à 6, dans lequel des parois internes de la première fente de mise en prise d'extrémité distale (131) et de la deuxième fente de mise en prise d'extrémité distale (132) sont rigides, dans lequel :
une direction d'ouverture de la première fente de mise en prise d'extrémité distale (131) est perpendiculaire à l'axe longitudinal et un angle inclus entre la direction d'ouverture et une direction radiale par rapport à l'axe est un angle aigu, une extrémité proximale (131b) de la première fente de mise en prise d'extrémité distale étant ouverte ; et
une direction d'ouverture de la deuxième fente de mise en prise d'extrémité distale (132) est au moins partiellement transversale à la première fente de mise en prise d'extrémité distale, et la deuxième fente de mise en prise d'extrémité distale est positionnée au niveau d'une extrémité radialement externe de l'aile.

8. Dispositif de fermeture de tissu selon l'une quelconque des revendications 1 à 7, dans lequel le mécanisme de limitation de profondeur comprend en outre un bloc de fixation (240) relié de manière fixe à l'extrémité distale du corps allongé et s'étendant le long de l'axe longitudinal, le bloc de fixation étant positionné de telle sorte que : les au moins deux ailes entourent circonférentiellement le bloc de fixation lorsque les au moins deux ailes sont dans la position pliée, et le bloc de fixation est situé de manière distale par rapport à une jonction des au moins deux ailes lorsque les au moins deux ailes sont dans la position dépliée,
dans lequel la première fente de mise en prise d'extrémité distale (241) est formée sur le bloc de fixation, et la deuxième fente de mise en prise d'extrémité distale (231) est formée sur les au moins deux ailes.

9. Dispositif de fermeture de tissu selon la revendication 8, dans lequel le bloc de fixation comprend une partie longitudinale (240a) s'étendant le long de l'axe longitudinal et une partie transversale (240b) perpendiculaire à l'axe longitudinal, la partie longitudinale et la partie transversale formant ensemble une unité cruciforme, dans lequel la première fente de mise en prise d'extrémité distale (241) est formée à deux extrémités de la partie transversale (240a).

10. Dispositif de fermeture de tissu selon la revendication 9, dans lequel la première fente de mise en prise d'extrémité distale (241) est formée sur une face distale au niveau d'une extrémité radialement externe de la partie transversale.

11. Dispositif de fermeture de tissu selon l'une quelconque des revendications 8 à 10, dans lequel la deuxième fente de mise en prise d'extrémité distale (231) est formée au niveau d'une extrémité distale de chacune des ailes.

12. Dispositif de fermeture de tissu selon l'une quelconque des revendications 8 à 11, dans lequel une extrémité distale de la partie longitudinale du bloc de fixation est en outre pourvue d'une troisième fente de mise en prise d'extrémité distale (242), la troisième fente de mise en prise d'extrémité distale étant conçue de telle sorte que la suture reste en prise avec la troisième fente de mise en prise lorsqu'elle est saisie par l'instrument.

13. Dispositif de fermeture de tissu selon l'une quelconque des revendications 7 à 12, dans lequel chaque aile est pourvue d'une fenêtre (232), la fenêtre étant positionnée pour permettre à la suture de s'étendre de manière distale à travers la fenêtre de l'évidement de réception de suture du corps allongé à la première fente de mise en prise d'extrémité distale.
